# FASCICULE DE BREVET EUROPEEN

(11) **EP 1 280 478 B1**
(45) Date de publication et mention de la délivrance du brevet: **31.03.2004**
(21) Numéro de dépôt: 01931800.5
(22) Date de dépôt: 04.05.2001
(51) Int. Cl.: A61F 2/00

(54) **PROTHESE DE REPARATION HERNIAIRE**
PROTHESE ZUR HERNIAREPARATUR
HERNIA REPAIR PROSTHESIS

(30) Priorité: 05.05.2000 FR 0005764
(43) Date de publication de la demande: 05.02.2003
(73) Titulaire: Cousin Biotech, 59117 Wervicq Sud (FR)
(72) Inventeur: DIEUDONNE, Gabriel, F-06800 Cagnes sur Mer (FR); FRISMAND, Jean, F-59700 Marcq en Baroeul (FR); SOLECKI, Gilles, 59390 Lys Lez Lannoy (FR)
(74) Mandataire: Thinat, Michel
(86) Numéro de dépôt international: PCT/FR2001/001370
(87) Numéro de publication internationale: WO 2001/085060

(56) Documents cités:
- EP-A- 0 898 944
- EP-A- 0 898 945
- WO-A-92/13500
- WO-A-97/45068
- FR-A- 2 778 554
- US-A- 5 569 273

## Description

La présente invention a pour objet une prothèse de réparation de hernies en particulier, mais non exclusivement, de hernies inguinales.

La paroi abdominale chez l'être humain est constituée de muscles et de graisse. Il arrive que cette paroi laisse passer le péritoine et les viscères qui forment alors une excroissance à la surface de la peau. On parle alors d'une hernie ombilicale ou inguinale en fonction de sa localisation.

De façon, à résoudre les problèmes concernant les hernies, les praticiens chirurgiens ont recours à différentes techniques chirurgicales pour refermer l'orifice herniaire :
- par fil de suture sous tension;
- par matériel prothétique en chirurgie ouverte avec un renfort de paroi sans tension tel que décrit par Stoppa, Rive, Lichtenstein ou Chastang ou utilisant un bouchon obturateur désigné couramment par« PLUG «;
- par coeliochirurgie : Mise en place de la prothèse de renfort en TEP ou en TAP.

Afin de réparer la paroi abdominale dans la région herniaire on implante chez l'homme un bouchon constitué de textile. Ce bouchon ou « plug » sert à éviter une récidive. Le bouchon obturateur selon l'invention est du type implanté par chirurgie ouverte mini invasive.

Jusqu'à maintenant, les praticiens utilisaient un bouchon en polymère assez massif (plug) composé de plusieurs couches de polymère avec une résistance radiale importante qui avait comme but initial d'occuper l'ensemble de l'espace de l'orifice. Cependant, dans ce cas, la masse implantée de polymère est très importante. Une fois colonisé par un tissu cicatriciel, celui-ci faisait vraiment bouchon et avait tendance à être expulsé par le phénomène de la pression abdominale vers l'extérieur. De plus, il y avait également possibilité de migration de cette masse importante et d'abrasion.

Le clocument WO 92/13500A décrit un dispositif de réparation pour hernie inguinale selon le préambule de la revendication 1.

Un premier objet de l'invention est de pallier les inconvénients et de proposer une prothèse dont le volume soit aussi restreint que possible afin d'éviter une trop grande quantité de produits étrangers dans l'organisme.

Selon l'invention, la prothèse de réparation pour hernie inguinale est caractérisée par le caractéristiques de la deuxième partie de la revendication 1.

Selon une autre caractéristique de l'invention, la collerette formée par le rebord est thermoformée. Ce rebord peut avoir une largeur de plusieurs millimètres et sert de butée sur la paroi abdominale.

De profil, l'implant rappelle la coque d'un navire et la base de cette partie est introduite dans l'orifice à combler. La base du plug est plate et horizontale s'opposant ainsi parfaitement à la poussée du sac herniaire. En vue par-dessus, la forme générale du bouchon apparaît ovale à partir du bord supérieur duquel fait saillie une collerette de quelques millimètres de tissu prothétique. Sa fixation particulière lui permet d'avoir un double effet : un effet bouchon par implantation de la partie conique du plug et un effet auto stabilisant, le maintien du bouchon étant réalisé par une suture superficielle grâce à la collerette et un effet prothèse en clé de voûte par amarrage du bouchon sur les tissus profonds. Cet amarrage est réalisé par quatre points en "U" noués à l'extérieur et par quatre points seulement posés sur la forme des droites en DD et l'arcade profonde en DH. Le passage de ces fils est possible du fait que le bouchon est creux. Il est facilité par le marquage d'une ligne de couleur visualisant le siège de passage du fil.

Le tronc de cône présentant deux arêtes courbes sert à obturer l'orifice de la paroi alors que la partie avant proéminente ou «bec de canard" permet une fixation aisée. En effet, dans le cas d'une hernie inguinale le chirurgien pratique une incision oblique dans l'aine. Il importe d'éviter que l'incision ne se prolonge à la suite de mouvements que le patient effectue naturellement, mouvements provoquant des tensions notamment sur les bords de l'ouverture et notamment dans la partie inférieure ou avant qui est faiblement musclée alors que la partie arrière l'est davantage. Le "bec de canard" renforce cette partie avant en la surplombant et en la protégeant pour éviter une déchirure de la paroi musculaire. Lors de la pose de l'implant, cette partie est suturée sur la paroi.

La prothèse est tricotée à larges mailles à partir d'une fibre synthétique biocompatible telle que du polypropylène, pour retenir la masse musculaire tout en permettant sa colonisation tissulaire. Sa configuration creuse et sa constitution à l'aide de deux couches de tissu prothétique lui assurent une certaine tenue en limitant la masse de tissu implanté.

La prothèse est une prothèse textile tricotée avec un monofilament de polypropylène ou en tout autre matériau biologiquement compatible. Avantageusement, elle peut être constituée de fils PGA ( acide polyglycolique) , PLLA (acide poly-L-lactique) ou leurs copolymères, présentant la propriété d'être résorbables à long terme. La pose de cette prothèse permet une chirurgie ambulatoire et peut se faire sous anesthésie locale ou loco-régionale.

La conception de la prothèse selon l'invention est différente des prothèses connues par sa forme anatomique qui est en parfaite osmose avec les différents orifices herniaires car elle vient s'adapter parfaitement aux parois de l'orifice.

Son centre n'est en aucun cas occupé par une masse importante de polymère inutile et donc ce produit à une masse de polymère, implantée à long terme dans le corps humain, nettement inférieure aux produits actuellement utilisés. Elle est souple et s'adapte parfaitement. Lorsque le patient tousse et exerce le maximum de pression abdominale, la prothèse se referme sur elle-même et se comporte comme un diaphragme venant clore l'orifice. Le plug étant formé, sa surface de contact avec l'organisme est réduite.

Sa partie intérieure évidée permet au chirurgien de bien visualiser sa position et de faire un ancrage par points de suture au sein même de la prothèse le long de la ligne de couleur créée de façon à repérer ces points.

Sa conception en forme de coque préformée lui donne une résistance exceptionnelle à la poussée abdominale et ne peut, par sa structure armée, en aucun cas, se déformer, s'écraser ou se comprimer.

Cette structure se comporte comme un tuteur de façon à ce qu'il y ait une fibrose cicatricielle de consolidation de l'implant.

Dans un mode de réalisation particulier, l'implant peut être soit semi-résorbable soit résorbable à long terme.

Les rebords sont thermoformés de façon à ce que le plan supérieur vienne se poser et ainsi consolider les bords de l'orifice herniaire. Une plaque de renfort complémentaire peut venir en surface.

Dans le cas des hernies inguinales de type "indirect", le plug est inséré dans l'orifice profond du canal inguinal et la partie "bec de canard" repose sur le pédicule épigastrique et le fascia transversalis jusqu'au pubis. Il pourra éventuellement renforcer ce dernier ce qui évite la nécessité d'une prothèse plate en superficie.

Dans le cas des hernies inguinales de type "direct", le plug est introduit au niveau de la brèche du Fascia Transversalis entre le pubis, en bas et le pédicule épigastrique en haut. Plus spécifiquement, dans ce type de hernie, la forme du bouchon ne désorganise pas l'architecture du canal inguinal. La partie "bec de canard" permet de combler un point faible existant souvent entre fascia transversalis et pubis, point faible, source de récidive.

D'autres caractéristiques et avantages de l'invention apparaîtront au cours de la description qui va suivre d'un mode particulier de réalisation, donné uniquement à titre d'exemple non limitatif, en regard des dessins qui représentent :
- la figure 1, une vue par-dessus d'une prothèse;
- La figure 2, la même prothèse vue en perspective;
- La figure 3, une vue de côté ;
- la figure 4, une vue par l'avant.

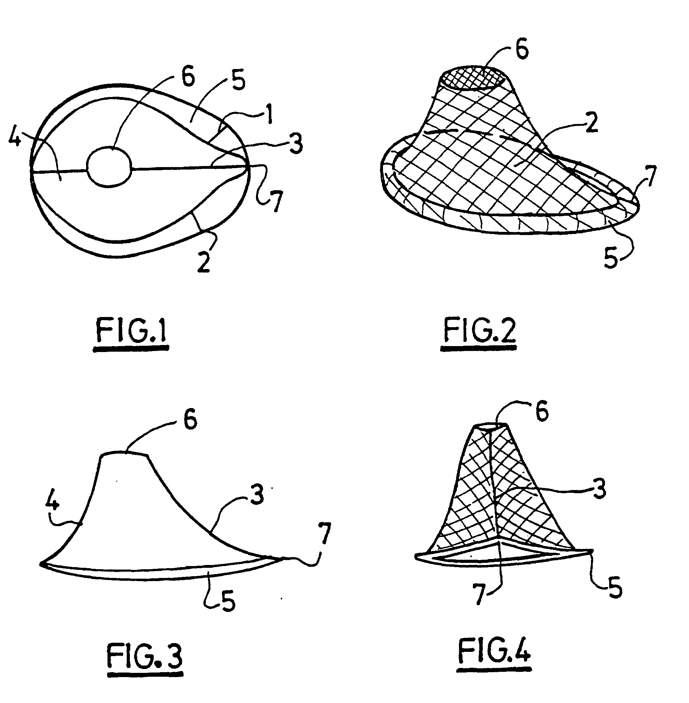

Sur l'ensemble des figures, les mêmes références désignent les mêmes éléments. Sur la figure 1, on voit que dans l'exemple représenté, la prothèse est constituée à partir de deux demi-patrons 1 et 2 qui sont assemblés bord sur bord au moyen d'une couture ou soudure avant 3 et arrière 4. Le fond 6 de l'implant résulte de la superposition de deux volets. Les deux parties 1 et 2 forment après assemblage une partie tronconique reliant la collerette 5 au fond 6. La collerette 5 est, de préférence, thermoformée de manière à lui conférer les caractéristiques d'élasticité nécessaires Elle repose sur le péritoine alors que la partie tronconique ou tétraédrique s'enfonce dans l'orifice à obturer.

Comme cela apparaît mieux sur la figure 3, la réalisation des coutures arrondit la collerette 5 qui repose sur la paroi abdominale de même forme. Les coutures 3 et 4 présentent des courbures différentes de sorte que la partie avant de l'implant soit proéminente par rapport à l'ensemble de celui-ci et forme un bec de canard analogue à une visière de chapeau.

Mais tout autre procédé de fabrication peut, bien entendu, être utilisé tel qu'un tricotage 3D qui évite la présence des coutures 3 et 4.

L'intérêt de l'implant selon l'invention peut se constater dans les interventions réalisées sous anesthésie locale. Lorsqu'un effort de toux est demandé au patient, on observe l'absence de poussée du bouchon, mais on note une rétraction de ses parois.

Il va de soi que de nombreuses variantes peuvent être apportées, notamment par substitution de moyens techniques équivalents, sans sortir pour cela du cadre de l'invention.

## Revendications

1. Prothèse de réparation pour hernie inguinale, comportant une partie creuse (1, 2) présentant une extrémité ouverte et un rebord (5), **caractérisé en ce que** la partie creuse est sensiblement tronconique (1, 2) et présente à l'autre extrémité un fond plat (6), **en ce que** le rebord forme une collerette (5) présentant une partie avant proéminente (7) s'étendant à partir de l'extrémité ouverte de la partie creuse, et **en ce que** la partie creuse présente, vue du dessus, une forme ovale définie par l'extrémité ouverte, et vue de côté un profil s'incurvant vers l'extérieur à partir du fond plat, ladite prothèse étant adaptée pour constituer une clé de voûte lors de son insertion dans des tissus.

2. Prothèse selon la revendication 1, **caractérisé en ce qu'**elle est tricotée à mailles larges à partir de fil synthétique biocompatible tel que du polypropylène.

3. Prothèse selon la revendication 1 ou 2, **caractérisé en ce qu'**elle est constituée par une double couche de tricot.

4. Prothèse selon la revendication 1, **caractérisé en ce qu'**elle est tricotée à mailles larges à partir de fil synthétique biocompatible résorbable en PGA, PLLA et leur copolymères.

5. Prothèse selon l'une quelconque des revendications précédentes, **caractérisé en ce que** la collerette (5) est thermoformée pour présenter une courbure.

6. Prothèse selon l'une quelconque des revendications précédentes, **caractérisé en ce qu'**elle est constituée par assemblage de deux demi-patrons par couture, collage ou soudure.

## Patentansprüche

1. Prothese zur Reparatur von Hernia inguinalis, umfassend einen hohlen Teil (1, 2), der ein offenes Ende aufweist, und eine Kante (5), **dadurch gekennzeichnet, dass** der hohle Teil etwa kegelstumpfartig (1, 2) ist und am anderen Ende einen flachen Boden (6) aufweist, dass die Kante einen Rand (5) ausbildet mit einem vorn vorspringenden Teil (7), der sich von dem offenen Ende des hohlen Teil aus erstreckt, und dadurch, dass der hohle Teil in der Draufsicht eine ovale Form aufweist, die vom offenen Ende definiert wird, und in der Seitenansicht ein Profil, dass sich vom flachen Boden ausgehend nach außen krümmt, wobei die besagte Prothese angepasst ist, um beim Einsetzen in Gewebe einen Abschluss zu bilden.

2. Prothese nach Anspruch 1, **dadurch gekennzeichnet, dass** sie aus biokompatiblem synthetischem Garn wie Polyprophylen weitmaschig gewirkt ist.

3. Prothese nach Anspruch 1 oder 2, **dadurch gekennzeichnet, dass** sie aus einer doppelt gewirkten Schicht besteht.

4. Prothese nach Anspruch 1, **dadurch gekennzeichnet, dass** sie aus biokompatiblem synthetischem, in PGA, PLLA und deren Copolymeren resorbierbarem Garn weitmaschig gewirkt ist.

5. Prothese nach einem der vorhergehenden Ansprüche, **dadurch gekennzeichnet, dass** der Rand (5) thermogeformt ist und eine Krümmung aufweist.

6. Prothese nach einem der vorhergehenden Ansprüche, **dadurch gekennzeichnet, dass** sie durch Montage zweier Halbschablonen durch Nähen, Kleben oder Schweißen gebildet wird.

## Claims

1. Inguinal hernia repair prosthesis comprising a hollow part (1,2) having one open end and an edge (5), **characterized in that** the hollow part is substantially of truncated cone shape (1,2) and at the other end has flat bottom part (6), **in that** the edge forms a rim (5) having a protruding front part (7) extending from the open end of the hollow part, and **in that** the hollow part, from an overhead view, is of oval shape defined by the open end and, from a side view, has a profile curving outwards from the flat bottom part, said prosthesis being adapted to form a keystone when inserted in tissues.

2. Prosthesis as in claim 1, **characterized in that** it is of wide-mesh knit in biocompatible synthetic yarn such as polypropylene.

3. Prosthesis as in claim 1 or 2, **characterized in that** it consists of a double layer of knit.

4. Prosthesis as in claim 1, **characterized in that** it is of wide-mesh knit in resorbable biocompatible synthetic yarn made of PGA, PLLA and their copolymers.

5. Prosthesis as in any of the preceding claims, **characterized in that** the rim (5) is thermo-moulded to have a curve.

6. Prosthesis as in any of the preceding claims, **characterized in that** it consists of two semi-patterns assembled by sewing, bonding or welding.
